# EUROPEAN PATENT SPECIFICATION

(11) **EP 3 758 846 B1**
(45) Date of publication and mention of the grant of the patent: **07.06.2023**
(21) Application number: 19710338.5
(22) Date of filing: 01.03.2019
(51) Int. Cl.: B01L 3/00, G01N 27/00

(54) **DEVICE FOR PERFORMING ELECTRICAL MEASUREMENTS**
VORRICHTUNG ZUR DURCHFÜHRUNG ELEKTRISCHER MESSUNGEN
DISPOSITIF PERMETTANT D'EFFECTUER DES MESURES ÉLECTRIQUES

(30) Priority: 02.03.2018 NL 2020518
(43) Date of publication of application: 06.01.2021
(73) Proprietor: Mimetas B.V., 2342 DH Oegstgeest (NL)
(72) Inventor: VULTO, Paul, 2342 DH Oegstgeest (NL); TRIETSCH, Sebastiaan Johannes, 2342 DH Oegstgeest (NL); NICOLAS, Arnaud Yannick Michel, 2342 DH Oegstgeest (NL); SCHAVEMAKER, Frederik Mathijs, 2342 DH Oegstgeest (NL)
(74) Representative: Haseltine Lake Kempner LLP
(86) International application number: PCT/EP2019/055187
(87) International publication number: WO 2019/166644

(56) References cited:
- WO-A1-2015/021993
- WO-A1-2016/046301
- US-A1- 2010 099 094
- ANDREESCU S ET AL: "AUTONOMOUS MULTIELECTRODE SYSTEM FOR MONITORING THE INTERACTIONS OF ISOFLAVONOIDS WITH LUNG CANCER CELLS", ANALYTICAL CHEMISTRY, AMERICAN CHEMICAL SOCIETY, US, vol. 76, no. 8, 15 April 2004 (2004-04-15) , pages 2321-2330, XP001196797, ISSN: 0003-2700, DOI: 10.1021/AC035436M
- THOMAS S. MANN ET AL: "Microplate-Compatible Biamperometry Array for Parallel 48-Channel Amperometric or Coulometric Measurements", ANALYTICAL CHEMISTRY, vol. 80, no. 8, 15 March 2008 (2008-03-15) , pages 2988-2992, XP055504532, US ISSN: 0003-2700, DOI: 10.1021/ac7020486
- REITER S ET AL: "Redox modification of proteins using sequential-parallel electrochemistry in microtiter plates", THE ANALYST,, vol. 126, no. 11, 15 October 2001 (2001-10-15), pages 1912-1918, XP002381714, ISSN: 0003-2654, DOI: 10.1039/B105059C
- SU GEORGE ET AL: "Effective treatment of mouse sepsis with an inhibitory antibody targeting integrin [alpha]v[beta]5", CRITICAL CARE MEDI, LIPPINCOTT WILLIAMS & WILKINS, US, vol. 41, no. 2, 1 February 2013 (2013-02-01), pages 546-553, XP009178690, ISSN: 1530-0293, DOI: 10.1097/CCM.0B013E3182711B1E
- VALERO T ET AL: "Studies on neuronal differentiation and signalling processes with a novel impedimetric biosensor", BIOSENSORS AND BIOELECTRONICS, ELSEVIER SCIENCE LTD. UK, AMSTERDAM, NL, vol. 26, no. 4, 15 December 2010 (2010-12-15), pages 1407-1413, XP027546856, ISSN: 0956-5663 [retrieved on 2010-07-23]
- OPP D ET AL: "Use of electric cell-substrate impedance sensing to assess in vitro cytotoxicity", BIOSENSORS AND BIOELECTRONICS, ELSEVIER SCIENCE LTD. UK, AMSTERDAM, NL, vol. 24, no. 8, 15 April 2009 (2009-04-15) , pages 2625-2629, XP026031413, ISSN: 0956-5663, DOI: 10.1016/J.BIOS.2009.01.015 [retrieved on 2009-01-23]
- TASEV DIMITAR ET AL: "CD34 expression modulates tube-forming capacity and barrier properties of peripheral blood-derived endothelial colony-forming cells (ECFCs)", ANGIOGENESIS, KLUWER, DORDRECHT, NL, vol. 19, no. 3, 4 April 2016 (2016-04-04), pages 325-338, XP035997205, ISSN: 0969-6970, DOI: 10.1007/S10456-016-9506-9 [retrieved on 2016-04-04]
- ANDREESCU ET AL: "Advanced electrochemical sensors for cell cancer monitoring", METHODS, ACADEMIC PRESS, NL, vol. 37, no. 1, 1 September 2005 (2005-09-01), pages 84-93, XP005109762, ISSN: 1046-2023, DOI: 10.1016/J.YMETH.2005.05.007

## Description

### Field of the Invention

The present invention relates to a device for performing electrical measurements, for example electrical activity across a layer of epithelial cells present in a microfluidic device. The present invention also relates to an *in vitro* method for measuring electrical properties of cells cultured in a microfluidic device, for example for determining the modulating effect of a test compound on epithelial barrier function.

### Background to the Invention

Epithelial tissue comprises one of the four basic tissue types (epithelial tissue, connective tissue, muscle tissue, and nervous tissue). Epithelial cells are found in animals (both in vertebrates and in invertebrates) as well as in plants and play a vital role in the physiology of the organism.

Epithelial cells line both the outside and the inside cavities and lumen of bodies. Endothelium (the inner lining of blood vessels, the heart, and lymphatic vessels) and mesothelium (forming the walls of the pericardium, pleurae, and peritoneum) are a specialized form of epithelium.

Epithelial cells form epithelial barriers that act as guards to the internal body. The cells and the barriers they form segregate the internal and external cavities of the body and provide a means for the body to selectively absorb and excrete particular substances. The epithelial barriers and the epithelial cells are for that reason important in a variety of biological processes, such as chemical and nutrient absorption, transcellular transport, detection of sensation, waste excretion, and protecting against microbial infection. All epithelia are usually separated from underlying tissues by an extra cellular fibrous basement membrane.

As an example, epithelia form the structure of the lung, including the alveoli or air sacs, and line most organs, such as the stomach and small intestine, kidney, and pancreas. They also line the esophagus and are found in ducts and glands, like the bile duct and salivary glands. They form taste buds, line the nose, the ear and the eye and the skin. The endothelium is the thin layer of endothelial cells that lines the interior surface of blood vessels and lymphatic vessels, forming an interface between circulating blood or lymph in the lumen and the rest of the vessel wall and underlying tissue. An example of this interface is the blood-brain barrier.

Mesothelial cells form a monolayer of specialized pavement-like cells that line the body's serous cavities and internal organs. The primary function of this layer, termed the mesothelium, is to provide a slippery, non-adhesive and protective surface. However, mesothelial cells play other pivotal roles involving transport of fluid and cells across the serosal cavities, antigen presentation, inflammation and tissue repair, coagulation and fibrinolysis and tumor cell adhesion.

Epithelial cells are characterized by a number of distinguishable characteristics. Epithelial cells are bound together in sheets of tissue called epithelia. These sheets are held together through several types of interactions, including tight junctions, adherens, desmosomes, and gap junctions. Tight junctions, or *zonulae occludentes,* act as the delineation between the apical (upper) and basal (lower) regions of an epithelial cell in conjunction with polarization between the two regions. Epithelium is supported on the basal side by a basement membrane called the basal lamina.

As mentioned, one distinguishing feature is the formation of tight junctions that segregate the plasma membrane of the polarized epithelial cell into an apical and a basolateral portion. The apical portion of the cell is the exposed, or top, portion of the cell when oriented in a cell monolayer grown *in vitro,* for example on a tissue culture plate. In the context of an epithelial cell sheet in the body, the apical surface would be exposed to the lumen lined by the epithelium. The basolateral surface of the cell is composed of the bottom, or basal, portion and the side, or lateral, portions. In the context of a cell grown on a tissue culture plate, the basolateral membrane of the cell is the portion of the cell contacting the tissue culture plate and the lateral portion of the cell situated below the tight junctions. In the context of an epithelial cell sheet in the body, the basolateral surface of the cell would be exposed to the internal portion of the body lined by the epithelium. Various proteins localize specifically to the apical or basolateral membrane. Given the importance it is not surprising that epithelial cells (including endothelial cells and mesothelial cells) are widely used to study a variety of biological processes. The cells are well suited for studies in fields like molecular cell biology, (microbial) pathogenesis, pharmacology, and toxicology.

Numerous model systems have been developed to study epithelial cells and barrier function. Studying epithelial cells normally requires the ability to access or modify the culture medium that is in contact with the apical or basolateral surfaces of the epithelial cells. Since standard tissue culture devices do not allow for this sort of manipulation specialized cell culture devices have been developed. The primary device used in most *in vitro* model systems is a permeable tissue culture plate insert, such as a Transwell^{®} (Corning, Inc., Lowell, Mass.). These devices provide an artificial permeable growth support that can be inserted into a well of a tissue culture plate. By culturing a polarized cell monolayer across the surface of the permeable growth support it will function as a selective barrier to separate the apical and basolateral chambers of the tissue culture well.

Such model systems play a vital role in the development of new medicines, understanding various diseases and understanding the toxic effects of agents.

For example, during the drug development process, potential therapeutic agents or drug candidates must be demonstrated to be both safe and effective for their intended use prior to obtaining approval and subsequent commercialization. Various drugs are known to negatively modulate epithelial barrier functions (see, e.g., Youmba et al. J Pediatr Gastroenterol Nutr 2012;54:463-70). On the other hand, compounds that modulate the barrier function of epithelial cells, for example by temporarily opening the barrier may be useful to improve drug delivery to the systemic circulation and to organs (Deli, Biochimica et Biophysica Acta - Biomembranes 1788 (4) 2009, 892-910).

Likewise, temporarily opening the blood brain barrier may be useful in delivery of drugs to the brain. Furthermore, such systems are important to understand the effect of all kinds of compounds, including those found in food, cosmetics, and beverages, and bacteria, on the barrier function. For example, Clostridium difficile toxins disrupt epithelial barrier function by altering membrane microdomain localization of tight junction proteins (Nusrat et al. Infect Immun. 2001 Mar;69(3):1329-36.), whereas other components may be increasing or supplementing epithelial barrier function.

While current epithelial cell model systems are useful for drug discovery, working with the cells in these systems has turned out to be difficult due to the highly uniform cell monolayers needed for this work. The experimental work requires choosing the correct cell type, producing multiple uniform cell monolayers, and ensuring cell monolayer integrity is sufficient to conduct the experiments. Furthermore, all of these must be well-established to allow for repeated production of experimentally acceptable results. These difficulties can make developing a desirable epithelial cell model system a daunting process, requiring months or years of work.

Devices generally directed to performing multiple, simultaneous electrical measurements in microtiter plates, are known in the art, for example as described in Andreescu S. et al., Analytical Chemistry 2004, 76(8), 2321; Thomas S. Mann et al, Analytical Chemistry, 2008, 80(8), 2988; Reiter S. et al., Analyst, 2001, 126(11), 1912; Andreescu S. et al., Methods, 2005, 37(1), 84; and US 2010/099094.

However, these devices are not suited for application in high throughput screening as it takes time to switch consecutive plates and to setup a measurement. There is great interest in the development of new high throughput screening devices and methods which are capable of rapidly providing data on electrical properties for a large number of different compounds. It is therefore an object of the present invention to provide an improved device and method which results in better understanding of the effects of compounds on electrical properties, for example on epithelial barrier function.

### Summary of the Invention

The present invention is defined in and by the appended claims. Thus, according to a first aspect of the present invention, there is provided a device for performing electrical measurements, comprising:
a cassette having first and second surfaces, the cassette configured to engage with a microtiter plate and comprising a plurality of electrodes extending from the first surface in the direction of the microtiter plate when the cassette is engaged with the microtiter plate; and
a housing detachably attached to the second surface of the cassette, the housing comprising one or more heat management elements, and a processor comprising a data acquisition module electrically connected to the electrodes and a data processing module.

A device according to the present invention enables use of the electrode cassette with a standard microtiter plate, without the need for electrodes or conductive surfaces integrated in the microtiter plate. This is made possible by having all electrodes for the electrical measurement in the electrode cassette and extending from a single surface of the electrode cassette for insertion into the microtiter plate. The device can be used with Transwell^{®} plates and Organoplates^{®} and does away with the need for a specialised titerplate comprising its own set of electrodes in its base. Cassettes can be configured to match the well layout of any titerplate, including the height and spacing of the wells as well as the possible connectivity between wells. The length of the electrodes can be configured to match the depth of the wells and/or to measure at different positions within the well. The cassette can also be configured to measure at multiple positions inside a single well, e.g. inside and outside a transwell insert present in a single well.

The terms 'cassette' and 'electrode cassette' are used interchangeably and have the same meaning throughout the specification. Similarly, the terms 'microtiter plate' and 'titer plate' are to be used interchangeably.

According to a second aspect of the present invention, there is provided an *in vitro* method for measuring electrical properties of a layer of cells cultured in a microfluidic device, the method comprising the steps of
a. providing a microfluidic device comprising a plurality of microfluidic channels, wherein at least one of the microfluidic channels is filled at least in part with a gel; and wherein at least one of the microfluidic channels comprises cells as a layer on or against the gel with an apical and a basolateral side, preferably the layer of cells having a tubular structure with an apical and a basolateral side in the microfluidic channel;
b. providing to the microfluidic channels at least one electrode in connection with the fluid in contact with the apical side and at least one electrode in connection with the fluid in contact with the basolateral side; thus incorporating the microfluidic channel in the electrical circuit;
c. measuring the impedance spectrum, voltage or current, wherein the microfluidic device is a microtiter plate, and
wherein the method is performed using a device according to the first aspect.

A device in accordance with the first aspect allows the electrode cassette to be detached from the data acquisition and processing electronics of the housing and to be cleaned.

### Brief Description of the Figures

The present invention will now be described by way of example only, with reference to the Figures, in which:
Figure 1 shows a device in accordance with the present disclosure;
Figure 2 shows an example microfluidic network including six electrode pairs for measuring electrical activity across the microfluidic network;
Figure 3 shows a further example of a microfluidic network for measuring electrical activity across the microfluidic network;
Figure 4 shows a cross-sectional and zoomed view of the electrodes of an electrode cassette being immersed in the culture medium inside a microfluidic channel;
Figure 5 shows a symmetrical and asymmetrical configuration for measuring electrical activity across the microfluidic network;
Figure 6 shows a typical impedance spectrum measured with a device and/or a method according to the invention;
Figure 7 shows the evolution of the TEER in time for a cultured tubule comprising Caco-2 cells; and
Figure 8 shows the effect of staurosporine on the TEER of Caco-2 tubules.

With specific reference to the Figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention.

### Detailed Description of the Invention

### Definitions

Various terms relating to the devices and methods of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

"A," "an," and "the": these singular form terms include plural referents unless the content clearly dictates otherwise. Thus, for example, reference to "a cell" includes a combination of two or more cells, and the like.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

"Comprising": this term is construed as being inclusive and open ended, and not exclusive. Specifically, the term and variations thereof mean the specified features, steps or components are included. These terms are not to be interpreted to exclude the presence of other features, steps or components.

"Exemplary": this term means "serving as an example, instance, or illustration," and should not be construed as excluding other configurations disclosed herein.

"Microfluidic system": this term refers to a device, or a fluidic component of a device, that is configured for containing, flowing, processing, or otherwise manipulating small volumes of liquid, such as in the sub-picoliter to sub- milliliter, or milliliter range. In some example embodiments, the maximal cross- sectional dimension of a microfluidic feature, such as a microfluidic channel, may be less than 1 mm, less than 500 microns, less than 100 microns, less than 50 microns, or less than 25 microns. Numerous microfluidic systems, devices, methods and manufacturing are known, including patent documents such as WO 2008/079320, WO 2013/151616, WO 2010/086179, WO2012/120101, or as commercially available from, for example, Mimetas, Leiden, The Netherlands (e.g. OrganoPlate; www.mimetas.com). While no particular limitations should be read from those applications and documents into any claims presented herein, these documents provide useful background material.

### Device

A device for performing electrical measurements is described. The device comprises a cassette having first and second surfaces, the cassette configured to engage with a microtiter plate and comprising a plurality of electrodes extending from the first surface in the direction of the microtiter plate when the cassette is engaged with the microtiter plate; and a housing detachably attached to the second surface of the cassette, the housing comprising one or more heat management elements, and a processor comprising a data acquisition module electrically connected to the electrodes and a data processing module.

In use, when the electrode cassette is engaged with a microtiter plate, the housing may be adjacent to the electrode cassette.

In some examples, the device is configured for impedance spectroscopy, potentiometry, voltammetry or amperometry. For example, the device may be configured for measuring transepithelial or transendothelial electrical resistance (TEER). The transepithelial or transendoethelial electrical resistance may be of a layer of cells in a microfluidic network within the microtiter plate. As another example, the device may be configured to be used as an Ussing chamber.

The different components of the device will now be described.

### Cassette

In one example, the cassette of the device is termed an electrode cassette. The cassette has first and second surfaces, with a plurality of electrodes extending from the first surface. In some examples, the second surface of the cassette is the opposite surface to the first surface. In some examples, the second surface of the cassette is a surface perpendicular to and adjoining the first surface.

The cassette is configured to engage with a microtiter plate in such a manner that the electrodes extend toward the microtiter plate when the cassette and microtiter plate are engaged with one another. The plurality of electrodes may be grouped into one or more subsets, each subset comprising at least two electrodes, for example at least three, for example at least four, for example at least five, for example at least six, for example at least seven, for example at least eight electrodes.

In some examples, the plurality of electrodes and/or the one or more subsets of electrodes are disposed in a predetermined configuration corresponding to the configuration of at least two or more wells of a microtiter plate; wherein the microtiter plate preferably comprises 96 microfluidic chips and wherein the microtiter plate preferably is a 384 well plate complying to the ANSI SLAS standards 1 to 4-2004. Thus, the electrode cassette may be manufactured so as to be compatible with a commercially available microtiter plate such as the Transwell^{®} or OrganoPlate^{®} titer plates.

For example, the configuration of the electrodes may be such that at least one subset of the electrodes is configured to correspond to at least one subset of wells that are microfluidically connected in the microtiter plate.

The electrodes, i.e. the plurality of electrodes, are configured to be immersed in a fluid inside the wells, thus incorporating the fluid in the electrical circuit. For example, the plurality of electrodes are of a length sufficient to extend from the cassette and into the wells of a microtiter plate engaged with the cassette. In this way, a fluid present in the well during electrical measurement completes the electrical circuit for any given subset of electrodes.

In some examples, each subset of electrodes contains at least a load or working electrode, a sense electrode and a reference electrode. In some examples, each subset of electrodes contains two or more electrodes that are directly connected in the electrical circuit. The two or more electrodes may be connected to one or more wells of the same microfluidic channel of a microtiter plate, so as to reduce the effective electrical resistance of the channel. In some examples, 2 or more of said subset of electrodes are configured such that the electrical circuit, formed when the cassette is engaged with the microtiter plate and a fluid is present in the microtiter plate, has similar electrical resistance across the directly connected electrodes. In this way the effect of the position of local differences in electrical characteristics on the apparent electrical characteristics of the entire system can be minimized. For example, when measuring the resistance across a locally disrupted cell layer separating two microfluidic channels with electrodes connected to only the proximal end of both microfluidic channels, the measured value will depend on how close the disruption is to the proximal end of said channels. If the disruption is closer to the electrodes, a lower overall resistance will be measured than if the disruption is further away from the electrodes, because more of the microfluidic channel can be at least partially bypassed.

When symmetrically connecting two short circuited electrodes to a proximal and distal end of a microfluidic channel in contact with the basal side of a cell layer, and symmetrically connecting two short circuited electrodes to a proximal and distal end of a microfluidic channel in contact with the apical side of a cell layer, and subsequently measuring across the cell layer, the position of any local disruption of said cell layer will have a reduced effect on the parameters measured.

To further illustrate this effect, both the apical and basal microfluidic channel can be viewed as a series of serially connected resistors. The cells and/or tight junctions between the cells, separating the two channels, can be viewed as parallel resistors connecting the apical and basal microfluidic channel. If the cells show high barrier function, the correlated resistor can be viewed as having a high resistance, while a local disruption of the cells or junctions can be viewed as a lowering of the resistance of said resistor. It can thus be understood that the position of the resistor with lowered resistance has more effect on the equivalent resistance of the circuit of an asymmetric circuit, than is the case in a symmetric circuit.

In further examples, the electrodes or electrode pairs connected to different ends of the microfluidic channels are not short circuited but are used to measure the electrical characteristics of said channels separately. By first characterizing the microfluidic channels in contact with the cells, and subsequently characterizing the circuit comprising cells as well as microfluidic channels, one can better determine the characteristics of the cells themselves.

In some examples, the plurality of electrodes and/or the one or more subsets of electrodes are disposed in a predetermined configuration corresponding to a single configuration of the microtiter plate. In other examples, the plurality of electrodes and/or the one or more subsets of electrodes are disposed in a predetermined configuration to optimize compatibility with a plurality of microtiter plate configurations. Compatibility with different titerplate configurations can be achieved by designing a layout of electrodes that is compatible with multiple plate layouts, or by electrically or otherwise switching the connectivity of one or more electrodes to adapt to different configurations of titer plates, or by adjusting the orientation of the cassette relative to the titer plate to adjust to different configurations of titer plates.

In some examples, the intended orientation of the cassette relative to the microtiter plate and/or the intended orientation of the cassette relative to the processor is secured by markings denominating the intended orientation, and/or by geometrical features that block engaging the multiple parts in any other position than the intended position. These features can include asymmetric registration pins, slots or similar features, and/or asymmetric chamfered corners.

In some examples two or more electrodes are immersed into a single well of a microtiter plate. This configuration allows for a 4-point electrical measurement which enables better electrical characterization of the electrical circuit and/or device under test (DUT), including elimination of the effect of double layer capacitance of the sensing electrode. This effect could be achieved by using one electrode to carry the majority of the current, while the other electrode is used for sensing. This reduces the polarization of the sensing electrode and thus largely avoids the formation of a double layer, which would otherwise impair low frequency impedance measurements. Further, a 4-point measurement reduces the influence of localized phenomena in the microfluidic channel, i.e. phenomena such as, but not limited to, changes in temperature, medium conductance, electrode position and the presence of air bubbles. These non-biological factors influence the channel resistance, thereby influencing the actual impedance read-out, which in turn makes it more difficult to derive the TEER value from the impedance spectrum.

The surface area and thickness of the electrodes is limited by the dimensions of the wells of the microtiter plate. Typically, the surface area is such that the resistance is negligible, irrespective of the medium level and contents in the wells. Any build-up of double layer capacitance is countered for by the 4-point measurement.

In some examples, the electrode material comprises a biocompatible material. For example, the electrode material may comprise platinum, gold plated brass, gold plated stainless steel or stainless steel. In other examples, the electrodes comprise one or more of a silver chloride electrode, an ion selective electrode, or a biofunctionalized electrode. In some examples the electrode material comprises a material that is resistant to degradation in or fouling by the solution it is intended to be immersed in. Such electrode materials are preferably highly noble, inert and/or corrosion resistant, such as gold, platinum or stainless steel. When the electrode material is stainless steel, it is preferably austenitic stainless steel, more preferably SAE type 316 stainless steel, more preferably SAE type 316F (food grade).

In some examples, the electrode cassette comprises at least 80 electrodes, more preferably at least 96 electrodes, more preferably at least 128 electrodes, more preferably at least 248 electrodes, more preferably at least 768 electrodes.

In some examples, the plurality of electrodes extends from the first surface of the cassette in a substantially or approximately parallel orientation to each other. It will be understood that this is not to be interpreted as requiring each and every electrode be straight and extend from the cassette at right angles to the first surface of the cassette. Instead, it will be understood that one or more electrodes may have a particular shape or configuration in order to allow it to engage with a particular well of a particular configuration of microtiter plate. In some examples subsets of the electrodes have different shapes or lengths to accommodate insertion in different positions within wells or in different wells.

In some examples, the plurality of electrodes is electrically connected to an electronics board, for example a Printed Circuit Board (PCB) housed within the cassette. In the context of the present invention, such an electronics board connected to the electrodes may also be referred to as an electrode board. The electrode board may have electrical connectors to allow it to be electrically connected to at least the data acquisition module of the housing. In some examples, the electrode board only comprises passive electrodes and conductive leads required to form the electrical circuits. In other examples the electrode board also comprises active electrical components, possibly including switches, multiplexers, amplifiers, and/or filters. The electrode board may further comprise a calibration PCB to calibrate the measurement electronics in the housing. The electrode board may further comprise a chip to store information such as serial numbers and calibration data.

In some examples, the electrode cassette comprises a casing in which the electrode board is located. The casing may be formed from any material typically used in laboratory devices, for example polycarbonate, polyethylene, polystyrene, polyoxymethylene, polytetrafluoroethylene, polyurethane, acrylate polymers, fiberglass, aluminium, stainless steel, or other plastics or metals. In some examples, the electrode cassette, including the plurality of electrodes, is compatible with laboratory cleaning techniques, formulations and equipment. For example, the electrode cassette may be compatible with acidic, basic, organic or inorganic cleansing solutions or oxidative cleansing solutions, or detergents and/or antiseptic solutions. The electrode cassette may further be compatible with ultrasound, autoclaving, sterilization by gamma irradiation, e-beam irradiation, ion beam irradiation, UV irradiation, ethylene oxide sterilization and/or (hydrogen peroxide) gas plasma sterilization. In some examples, the electrode board is removable from the electrode cassette. In other examples, the electrode board is fixed within the electrode cassette. In some examples, since the electrode board and/or electrode cassette are interchangeable depending on the microtiter plate being used, the electrode board and/or electrode cassette may be reused (after appropriate cleaning), and/or considered as a laboratory consumable.

In some examples the outer dimensions of the cassette extend beyond the outer dimensions of the electrodes and/or electrode board, ensuring that the cassette can be placed on a surface without the any of the electrodes touching said surface. In addition, this embodiment helps increasing the tortuous path sterility, i.e. it is easier to keep the microtiter plate that is attached to the cassette sterile. Under these conditions, gas exchange still occurs, allowing respiration of the cells under measurement. In further examples, the shape of the cassette is such that it helps guide the electrodes and/or connectors to their intended position during interfacing with the titer plate and/or housing. Such guiding action minimizes the risk of damage to any of the components during operation and improves ease of use.

Having a cassette that is easily attached to and detached from the housing, allows for the use of one housing with multiple cassettes. This is advantageous when using the device in a high throughput environment.

When the cassette is engaged with a microtiter plate, the whole combination can be disengaged from the housing. Leaving the cassette attached to the microtiter plate makes it possible to take a sterile plate out of an incubator and use it in a non-sterile environment, e.g. in the TEER device. The microtiter plate and its contents will remain sterile, even after measurement. It can be placed back in the incubator to perform a measurement at a later time point. It is even possible to transfer the cassette-microtiter plate combination to a different laboratory.

Another option made possible by having the components detachably detached is to use different cassettes with one microtiter plate. Those cassettes may have different electrode configurations for different purposes, e.g. configurations for amperometry, pH measurement and O₂ sensing.

### Housing

The housing of the device may be configured to be detachably attached to the electrode cassette, for example the second surface of the electrode cassette. In this manner, a surface of the housing may be in direct contact, or in close proximity to the second surface of the electrode cassette, meaning that the device has a small footprint, thus increasing the portability of the device. This portability makes the device suitable for use in an incubator and suitable for combination with a laboratory rocker, which is advantageous in a high throughput environment. In other examples, the housing may be detachably attached to the electrode cassette by means of an electrical connection only. In this manner, the housing (which houses the electronic circuitry) could be placed adjacent to the electrode cassette during use, or even spaced further away. Having the cassette and housing in close proximity also reduces the build-up of parasitic capacitance and reduces noise.

The shape of the housing may be configured in such a way that it can only be attached to the electrode cassette in a single orientation. In some embodiments of the invention, the mechanical attachment between the housing and electrode board is carried solely by the electrical connectors. In other embodiments, a dedicated mechanism is provided to clamp, lock, screw or otherwise reversibly fix the attached parts to each other.

The housing of the device may comprise one or more electronic boards a.k.a. Printed Circuit Board (PCB) to regulate and power the electronic components required to perform a measurement. The housing may further comprise a processor or processing unit disposed on one of the PCBs. Said processor can be implemented in numerous ways, with software and/or hardware, to control the electrical measurements, to acquire data and process the data. In particular implementations, the processor can comprise a plurality of software and/or hardware modules, each configured to perform, or that are for performing, individual or multiple steps of the measurement method. The processor may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to control the electrical input, perform the electrical measurements and process the data. The processor may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

In some examples, the processor comprises a controller, and the controller and/or processor control electrical input to the electrode board in order to measure electrical activity or electrical properties. In some examples, the processor comprises a data acquisition module electrically connected to the electrode board of the electrode cassette, and a data processing module. The data acquisition module is configured to acquire data from the plurality of electrodes in the form of electrical signals, while the data processing module is configured to process the data acquired by the data acquisition module.

In some examples, the data acquisition module is configured to acquire data based on the processor performing AC frequency sweeps, preferably in a range of from 0.1 Hz to 100 MHz, more preferably in a range of from 1 Hz to 100 MHz, more preferably in a range of from 10 Hz to 100 MHz, preferably in a range of from 10 Hz to 90 MHz, preferably in a range of from 10 Hz to 80 MHz, preferably in a range of from 10 Hz to 70 MHz, preferably in a range of from 10 Hz to 60 MHz, preferably in a range of from 10 Hz to 50 MHz, preferably in a range of from 10 Hz to 40 MHz, preferably in a range of from 10 Hz to 30 MHz, preferably in a range of from 10 Hz to 20 MHz, preferably in a range of from 10 Hz to 10 MHz. In some examples, the range of the frequency sweep and/or the frequency of the data collection is adaptable in a manual, automated or iterative fashion, preferably optimized to the characteristics of the system being measured. For instance, this means that a first measurement is performed over the full frequency range and that subsequently that measurement is used to adapt the frequency range, e.g. to zoom in on the TEER region of an impedance spectrum. This increases the measurement speed which is an important factor in the high throughput measurements that can be performed with a device and/or method according to the invention. It is to be understood that the application of AC frequency sweeps places special requirements on the heat management elements, as the alternating electrical field generates heat, especially in the higher frequency ranges, and especially in comparison with electrical devices that use DC equipment as a voltage source.

In some examples, the housing may comprise a memory, or may be configured to communicate with and/or connect to a memory external to (i.e. separate to or remote from) the housing. The memory may comprise any type of non-transitory machine-readable medium, such as cache or system memory including volatile and non-volatile computer memory such as random-access memory (RAM), static RAM (SRAM), dynamic RAM (DRAM), read-only memory (ROM), programmable ROM (PROM), erasable PROM (EPROM), and electrically erasable PROM (EEPROM). In some examples, the memory can be configured to store program code that can be executed by the processor of the housing to cause the processor to and perform the measurement protocol. Alternatively, or in addition, in some examples, the memory can be configured to store information resulting from or used in the method. For example, in some examples, the memory may be configured to store measurement protocols, including preset voltage and/or current amplitudes, and preset intervals for acquiring data, or any other information, or any combination of information, resulting from or used in the measurement method. The processor can be configured to control the memory to store information resulting from or used in the measurement method.

In some examples, the housing may comprise a user interface, or may be configured to communicate with and/or connect to a user interface external to (i.e. separate to or remote from) the housing. The user interface can be configured to render (or output, display, or provide) information resulting from or used in the measurement method. For example, in some examples, the user interface may be configured to render (or output, display, or provide) any one or more of an impedance spectrum, or voltage or current readouts at a time point or series of time points, or any other information, or any combination of information, resulting from or used in the measurement method. Alternatively, or in addition, the user interface can be configured to receive a user input. For example, the user interface may allow a user to manually enter information or instructions, interact with and/or control the device via the housing. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (e.g. on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some examples, the user interface that is controlled to render information may be the same user interface as that which enables the user to provide a user input. The processor can be configured to control the user interface to operate in the manner described herein.

In some embodiments, the housing may comprise a communications interface (or communications circuitry). The communications interface can be for enabling the device (or any components of the device, such as any component of the housing such as the processor, the memory, the user interface, and/or any other components of the housing) to communicate with and/or connect to one or more other components, such as other, interfaces, devices, memories, etc. The communications interface may enable the device (or any components of the device) to communicate and/or connect in any suitable way. For example, the communications interface may enable the device (or any components of the device as may be found in the housing) to communicate and/or connect wirelessly, via a wired connection, or via any other communication (or data transfer) mechanism. In some wireless embodiments, for example, the communications interface may enable the device (or any components of the device as may be found in the housing) to use radio frequency (RF), Bluetooth, or any other wireless communication technology to communicate and/or connect.

The device, in particular the housing, may comprise a battery or other power supply for powering the device or means for connecting the device to a mains power supply. It will also be understood that the device may comprise any other component to those described herein or any combination of components.

In some examples, the one or more heat management elements comprise elements thermally decoupling the cassette from the housing, such as insulating layers or spacers between the housing and cassette. For example, the one or more heat management elements may include passive or active heat conduits to move heat away from the cassette, wherein the one or more heat management elements comprise one or more of radiating surfaces such as a heat sink, cooling fins, liquid cooling, Peltier modules, air ducts, or fans improving airflow through or around the device, or any combination thereof. Managing, or minimising, heat transfer from the cassette to the housing, or from the housing to the cassette greatly improves the efficiency of the device and increases its lifespan. Managing the heat transfer is also important when performing measurements on cell cultures inside a microtiter plate, since the viability and behaviour of a cell depends strongly on the value and the stability of the temperature under which it is cultured.

In some examples heat transfer from the housing to the cassette is minimized by increasing the distance between the housing and the cassette, such as by including spacers between the housing and the cassette. In some examples this distance is used to further improve heat management, by imposing an airflow between the cassette and the housing. Further, in some examples convection is used to move heat away from the cassette. In some examples an airflow is imposed through the housing to carry heat out of the housing. In some examples this can be achieved by placing a fan in the housing that forces air into the housing and providing conduits for the warm air to exit the housing. Such a fan could be positioned on a top, bottom or side surface. The exhaust conduits can be configured to force air out of any surface of the housing, preferably facing away from the microtiter plate, more preferably facing in a direction where it is unlikely for other objects to be placed, potentially reducing the risk of cross contamination.

In some examples the material of the different components of the device is selected to improve heat transfer by conduction; away from the cassette while limiting conduction towards the cassette. Said material may include materials with low thermal conductivity between the cassette and the housing, and/or materials with high thermal conductivity on the sides of the housing facing away from the cassette.

When a heat sink is employed as a heat management element, or another passive heat management element comprising metal is employed, the heat sink may be grounded to prevent electrical noise disturbing the measurement and to prevent build-up of parasitic capacitance.

In a further example, a combination of heat management elements is incorporated in the device. In a certain embodiment, a passive heat sink is combined with a fan to force out the heat radiation emitted by said heat sink. In this embodiment the heat sink is on top of the electronics board comprising the processor and the fan is on top of the heat sink. On top here means the surface facing away from the cassette, thus directing away the induced heat from the electrode cassette by conduction and forced convection respectively.

In a further embodiment of the present device the housing comprises two PCBs. This allows for distributing the electronic components in such a way that, with respect to the cassette, the hot components, i.e. the components that generate the most heat, are on the top PCB. The top PCB is the PCB farthest away from the cassette when cassette and housing are detachably attached. As a consequence, the middle PCB, i.e. the PCB closest to the cassette, uses less power, thereby generating less heat, thereby minimizing heat convection or conduction in the vicinity of the electrode cassette. In this particular embodiment, the device comprises three PCBs. One is the electrode board in the cassette, the other two are comprised in the housing. The transfer of heat between the middle and top PCB is minimized by placing a isolating layer, e.g. a plastic layer, between both PCBs.

It is noted that the skilled person conveniently makes any useful combination of the heat management elements and measures described above.

In some examples, the device may further comprise a base configured to receive a microtiter plate and to detachably engage with the cassette and/or housing. The base may be configured to receive a microtiter plate and hold it securely, even in the absence of the cassette.

In some examples, the device may further comprise a clamping mechanism to ensure one or more of the following: fast, accurate and repeatable positioning of the cassette to the housing; fast, accurate and repeatable positioning of the electrodes within the wells of the microtiter plate; fast, accurate and repeatable positioning of the cassette and/or the housing to a base; fast, accurate and repeatable positioning of a microtiter plate to a base. The possibility of quick, accurate and repeatable connection and disconnection of the several components of the device further facilitates the use of the device in a high throughput screening environment.

In some examples, the total footprint of the device is less than twice the footprint of the titer plate, preferably less than 1.5 times the footprint of the microtiter plate, thereby allowing interaction of the microtiter plate with external equipment while engaged in the device.

### In vitro method for measuring barrier function of cells cultured in a microfluidic device

According to a second aspect, there is provided an *in vitro* method for measuring electrical properties of a layer of cells cultured in a microfluidic device, the method comprising the steps of
a. providing a microfluidic device comprising a plurality of microfluidic channels, wherein at least one of the microfluidic channels is filled at least in part with a gel; and wherein at least one of the microfluidic channels comprises cells as a layer on or against the gel with an apical and a basolateral side, preferably the layer of cells having a tubular structure with an apical and a basolateral side in the microfluidic channel;
b. providing to the microfluidic channels at least one electrode in connection with the fluid in contact with the apical side and at least one electrode in connection with the fluid in contact with the basolateral side; thus incorporating the microfluidic channel in the electrical circuit;
c. measuring the impedance spectrum, voltage or current, wherein the microfluidic device is a microtiter plate, and
wherein the method is performed using a device according to the first aspect.

In some examples, the microfluidic device is a microtiter plate. Numerous microfluidic systems, devices, methods of manufacturing are known, as well as methods for partly filling such devices with a gel and culturing cells so as to form tubular cellular structures on or against the gel with an apical and a basolateral side. Examples of such publications include WO 2008/079320, WO 2010/086179, WO 2012/120101, WO 2012/120102, WO 2013/151616, WO 2017/007325, WO 2017/155399, WO 2017/216113, with titerplates being commercially available from, for example, Mimetas, Leiden, The Netherlands (e.g. OrganoPlate^{®}; www.mimetas.com). While no particular limitations should be read from those applications and documents into any claims presented herein, these documents provide useful technical information on the provision of a microfluidic device in which at least one microfluidic channel is filled in part with a gel and comprises cells as a layer on or against the gel with an apical and a basolateral side.

In some examples, the microfluidic device comprises at least 40 channel networks, more preferably 64 channel networks, more preferably 96 networks. It will be understood that each channel network of the microfluidic device may have at least one microfluidic channel, for example at least two microfluidic channels in fluid communication with each other, for example at least three, for example at least four microfluidic channels in fluid communication with each other. It will be understood that the microfluidic channels of each network are preferably separated by means of capillary pressure techniques, such as pillars, ridges, groves, hydrophobic patches or less hydrophilic patches in a predominantly more hydrophilic channel, as described in the above-mentioned publications.

In some examples, the gel is a basement membrane extract, an extracellular matrix component, collagen, collagen I, collagen IV, fibronectin, laminin, vitronectin, D-lysine, entactin, heparan sulphide proteoglycans or combinations thereof. In some examples, the gel is in direct contact with the cell layer without any membrane separating the two. Such a system is enabled by the use of phaseguides or capillary pressure barriers in the microfluidic system as describe above. For example, the gel may be structured in the microfluidic channel through the use of such capillary pressure techniques. In this manner, in a multilane microfluidic network with the lanes or microfluidic channels separated by such capillary pressure barriers, a gel can be introduced to one channel and allowed to set. Once the gel is at least partially set, cells can be introduced into one or more adjacent lanes, and allowed to form a layer in contact with the gel. In some examples, the cells are endothelial or epithelial cells. In some examples, one or more additional cell types are co-cultured with the cells.

Through the use of a microfluidic device having a particular configuration of microfluidic networks and patterned gels therein, and wells for introducing or extracting liquids from the microfluidic channels, it is possible to selectively and accurately introduce electrodes, for example microelectrodes, into the microfluidic channels such that at least one electrode is in contact with a fluid contained in a microfluidic channel in contact with the apical side of a layer of cells, while simultaneously introducing at least one electrode into the microfluidic channels such that the at least one electrode is in contact with a fluid contained in a microfluidic channel in contact with the basolateral side of the layer of cells. In this manner, the microfluidic channel and its contents, specifically the gel, the layer of cells and fluids present in the microfluidic channel on the apical and basolateral sides of the cells) become part of an electrical circuit. Connection of the electrodes to a power source and data acquisition means, for example as part of a device as described herein, then allows measurement of electrical activity of the microfluidic network, in particular electrical activity across the layer of cells.

In some examples, measuring the electrical activity, or measuring electrical properties, comprises measuring the impedance spectrum, the voltage or the current of the electrical circuit comprising the layer of cells. For example, measuring the electrical activity may comprise taking measurements for impedance spectroscopy, potentiometry, voltammetry or amperometry. In one example, the method may comprise measuring transepithelial or transendothelial electrical resistance (TEER) of the layer of cells in the microfluidic network within the microtiter plate. Methods and protocols for measuring impedance, or transepithelial or transendothelial electrical resistance of a layer of cells, are known in the art, for example as described in WO 2004/010103, WO 2005/098423 and in van der Helm et al., Biosensors and Bioelectronics 85 (2016) 924-929.

In some examples, flow is induced through at least a subset of the microfluidic channels during the measurement. Inducing flow during measurement facilitates transport of media or test solutions through the microfluidic channels. In some examples, flow is induced by liquid levelling, preferably by reversibly tilting the microfluidic device under a specified angle and a specified time frame. For example, a microfluidic device can be tilted under an angle of 4 to 9°, preferably 5 to 7°. The period of the reversed tilting can be as short as 1 minute, but typically lies between 5 and 15 minutes.

In some examples, multiple cell layers and/or microfluidic channels are part of the same microfluidic network, and the impedance spectrum, voltage or current measured across said multiple cell layers occurs in a single, sequential or parallel measurement.

In some examples, all or part of the measurements in the plurality of microfluidic channels are being performed in parallel. In some examples, the measurement is performed multiple times to monitor the barrier function of the layer of cells over time. In some examples the electrical characteristics can be monitored for a short time to monitor acute changes, for example over the course of seconds, minutes or hours, performing measurements at intervals of less than a second, seconds, minutes or hours. In other examples the electrical characteristics can be monitored for prolonged periods of time to monitor slow or delayed changes, for example over the course of days, weeks or months, performing measurements at intervals of hours, days, weeks or months.

In some examples, the cultured cells are exposed to one or more compounds or other stimuli before or during the measurement, to observe the effect of said stimuli on the barrier function. The one or more compounds, or other stimuli which may be drug candidate compounds, can be introduced to the cultured cells via an inlet of the microfluidic network to a microchannel or part thereof adjacent to the layer of cells contacting the gel.

In some examples, the electrical measurements are performed in conjunction with other measurements, for example imaging and (bio-)chemical analysis.

In some examples the measurements are performed to characterize the system under test before other measurements or experiments are performed. In such an example, the measurements can be part of a quality control regime or any other setting that requires measuring the electrical characteristics without strongly influencing said system. These measurements could be referred to as non-invasive, minimally invasive, non-disruptive, minimally disruptive or non-destructive measurements.

In some examples, the method is performed using a device as described herein.

### Method for cleaning a device

The device of the invention may be cleaned in a cleaning method, the method comprising the steps of
(a) engaging the cassette with a cleaning plate comprising wells that receive the electrodes, the wells comprising a cleaning solution in which the electrodes are immersed;
(b) allowing the cleaning solution to remove any material build-up from the electrodes;
(c) optionally providing active actuation during cleaning, such as electrical, thermal, mechanical or acoustic actuation,
wherein the cleaning solution preferably comprises one or more of an acid, a base, an oxidizing agent, an organic solvent, a detergent or a disinfectant.

A suitable acid cleaning solution is a solution comprising one or more of acetic acid, sulphuric acid, nitric acid; a suitable base cleaning solution comprises sodium or potassium hydroxide; a suitable oxidising agent cleaning solution comprises one or more peroxides such as hydrogen peroxide, or sodium hypochlorite (bleach). Suitable organic solvents used for cleaning are an ethanol/water solution comprising at least 70 wt-% ethanol, acetone and isopropyl alcohol. Suitable detergents are plain dishwashing liquid, TWEEN and Triton-X. A suitable disinfectant would be chlorhexidine.

### Method for calibrating a device

The device of the invention may be calibrated in a method for calibrating a device, the method comprising the steps of
(a) engaging the cassette with a calibration plate such that the electrodes contact a reference system comprising a calibration solution and/or an electrical circuit;
(b) determine electrical characteristics of the electrodes and compare said characteristics with reference values;
(c) applying offset values or otherwise correcting the calibration of the device according to the measured characteristics.
(d) optionally cleaning the electrodes according to a method described herein.

Optionally, the electrode board in the cassette comprises a designated calibration PCB to calibrate the electronics in the housing.

### Kit of parts

The present disclosure also relates to a kit of parts comprising a cleaning plate comprising wells that can engage with the plurality of electrodes of the device of the invention, and one or more vials comprising a cleaning solution, the cleaning solution preferably comprising one or more of an acid, a base, an oxidizing agent, a reducing agent, an organic solvent, disinfectant or a detergent (not claimed).

The kit of parts may further comprise an actuator module providing actuation during cleaning, such as electrical, thermal, mechanical or acoustic actuation

### Detailed Description of the Figures

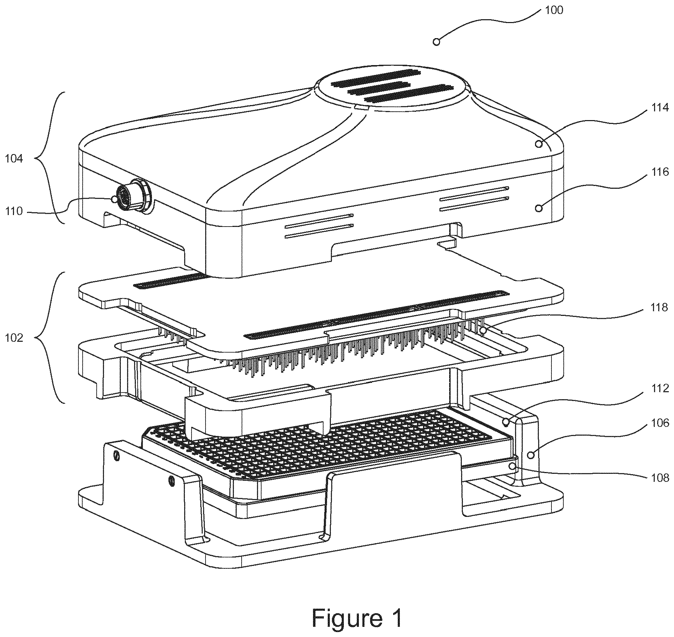

Figure 1 shows a device 100 in accordance with the present disclosure. Device 100 comprises an electrode cassette 102, comprising the plurality of electrodes 118, which extends from the lower surface of electrode cassette 102. Device 100 further comprises housing 104, which is configured to be detachably attached to electrode cassette 102. Housing 104 includes a heat management element 114, to manage heat transfer between electrode cassette 102 and housing 104 and the environment, and further comprises a processor or processing unit. To this end heat management element 114 is made of a metal, e.g. aluminum and equipped with air ducts to enable the flow of air. Not shown in the Figure, the housing 104 also comprises a heat management element 114 in the form of a fan, to enable air circulation. The housing further comprises a processor module 116 comprising the data acquisition module and data processing module described herein.

Figure 1 also shows optional base 106, which may be configured to detachably engage with electrode cassette 102 and/or housing 104. Also depicted in Figure 1 is microtiter plate 108, which is received by and housed in optional base 106, and with which electrode cassette is configured to engage. As has been described previously, base 106 is not essential to the functioning of the device and electrical measurements may be taken in its absence, due to electrode cassette 102 forming a secure engagement with microtiter plate 108 through a simple push fit or other clamping mechanism.

Also shown in Figure 1 is electrical/data connectivity port 110 for transmitting and receiving data obtained during electrical measurements to an external device, for example a display unit. Figure 1 also depicts clamping mechanism 112, in the form of a spring-loaded screw mechanism to secure housing 104 to at least electrode cassette 102.

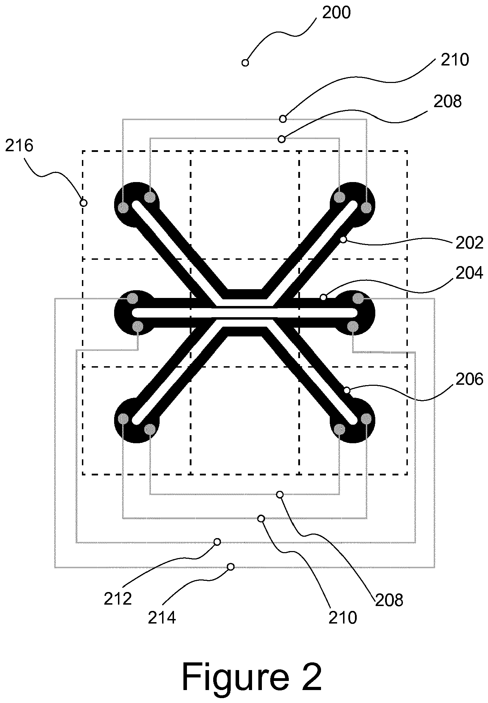

Figure 2 shows an example microfluidic network 200 including six electrode pairs for measuring electrical activity across the microfluidic network. Such a set up allows measurement of barrier function, e.g. by measuring transepithelial electrical resistance, of cells cultured in the microfluidic device in accordance with a method described herein and may be realised using a device as described herein.

In microfluidic network 200, which may be present in a microtiter plate, three lanes or microfluidic channels are present (202, 204 206). At one end of each microfluidic channel, an inlet can be seen, with a corresponding outlet at the other end of each microfluidic channel. The inlets and outlets of the microfluidic channels correspond to the well outline 216 of the microtiter plate. The boundaries between two microfluidic channels are defined by capillary pressure barriers as described previously (not shown). For example, at the central section of the microfluidic network where all three microfluidic channels or lanes come together, a capillary pressure barrier is present at the contact region between two microfluidic channels. As a result, a gel precursor solution can be introduced microfluidic channel 204. The gel precursor solution is then pinned by a capillary pressure barrier at the intersection with channel 200, and by a second capillary pressure barrier at the intersection with channel 206. After gelation of the gel precursor solution, culture media containing cells, for example epithelial cells can be introduced into lanes 202 and/or 206, enabling growth of a layer of cells on the gel present in lane 204. Once the layer of cells having an apical side and a basolateral side is established, the electrodes can be introduced into microfluidic network 200.

In Figure 2, six electrode pairs are introduced to microfluidic network 200, specifically reference electrodes 208 and counter electrodes 210 across each inlet/outlet of lanes or channels 202 and 206. In the embodiment depicted in this figure, the electrodes are all introduced from the top of the microtiter plate, for example via an electrode cassette as described previously.

The use of reference electrodes 208 and counter electrodes 210 in the configuration shown in Figure 2 reduces the effective electrical resistance of the channel, optimizing the resulting field homogeneity. Completing the measurement setup are working electrodes 212 and working sense electrodes 214 introduced into the inlet and outlet of microfluidic channel or lane 204 containing the gel and layer of epithelial cells. It will be understood that each of the electrodes depicted may be independently electrically connected to an electrode board, which may furthermore be connected to a processor configured to control power supply to the electrodes and to control data acquisition and processing of acquired data, as described previously.

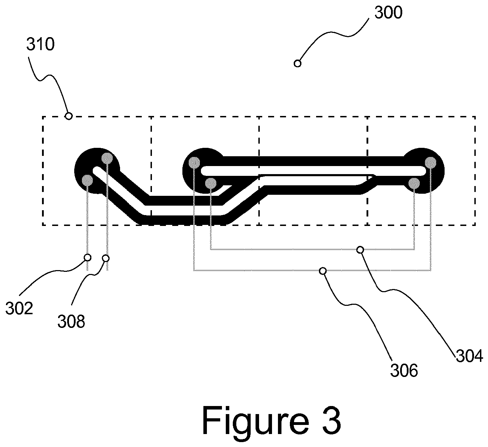

In a similar fashion as in Figure 2, Figure 3 shows a microfluidic network 300 in which a counter electrode 302, reference electrodes 304, working electrodes 306 and a sensing electrode 308 are used for measurement in the configuration shown.

It will be understood that the configurations of Figures 2 and 3 are illustrative examples only, and that a different number of microfluidic channels could be used, and the number of electrodes or electrode pairs may vary, depending on the nature of the electrical measurement.

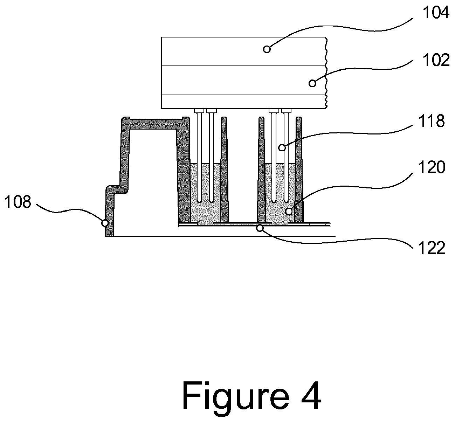

Figure 4 shows a schematic cross-section of the device according to the invention, the device being in measuring mode. The electrode cassette 102, being detachably engaged with housing 104, is detachably engaged with a microtiter plate 108, of which a single microfluidic channel 122 is shown. The electrodes 118 are immersed in the culture medium 120 inside the wells of the microfluidic channel. By immersing the electrodes in the culture medium, the electrical circuit is closed.

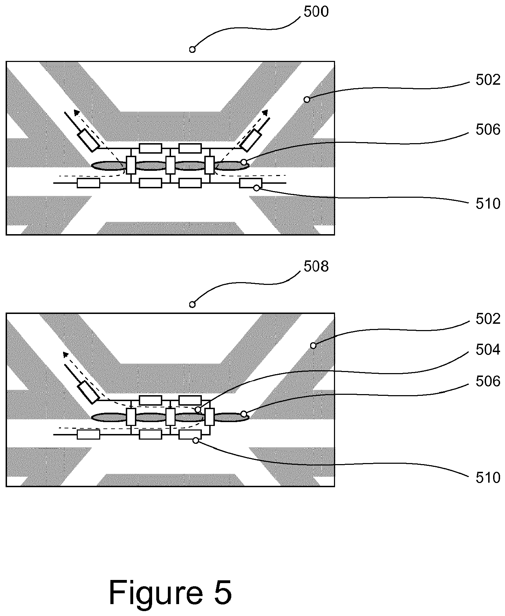

Figure 5 shows two possible measurement configurations, a symmetrical measurement configuration 500 and an asymmetrical measurement configuration 508. The symmetrical configuration 500 uses four pairs of measurement electrodes (not shown) positioned in the inlet and outlet wells (not shown) of the microfluidic channels 502 of a microfluidic chip. The asymmetrical configuration 508 uses two pairs of electrodes positioned in the wells on one side of the microfluidic chip, e.g. the left side in this Figure. Further shown are cells 506 forming a layer and resistances 510. The resistances or resistors 510 in the channels 502 running parallel to the layer of cells 506 can be viewed as a series of serially connected resistors 510. The cells 506 and/or tight junctions between the cells, separating the two channels 502, can be viewed as parallel resistors 510. This symmetric configuration is the preferred configuration because it minimizes or even eliminates the effect of the position of local differences in electrical characteristics on the apparent electrical characteristics of the entire system. For example, when measuring the resistance 510 across a locally disrupted cell layer separating two microfluidic channels 502 with electrodes connected to only the proximal end of both microfluidic channels 502, the measured value will depend on how close the disruption is to the proximal end of said channels. If the disruption is closer to the electrodes, a lower overall resistance will be measured than if the disruption is further away from the electrodes, because more of the microfluidic channel can be at least partially bypassed. The difference between the symmetrical and asymmetrical configuration can be illustrated by showing the flow of current 504 through the microfluidic channels 502.

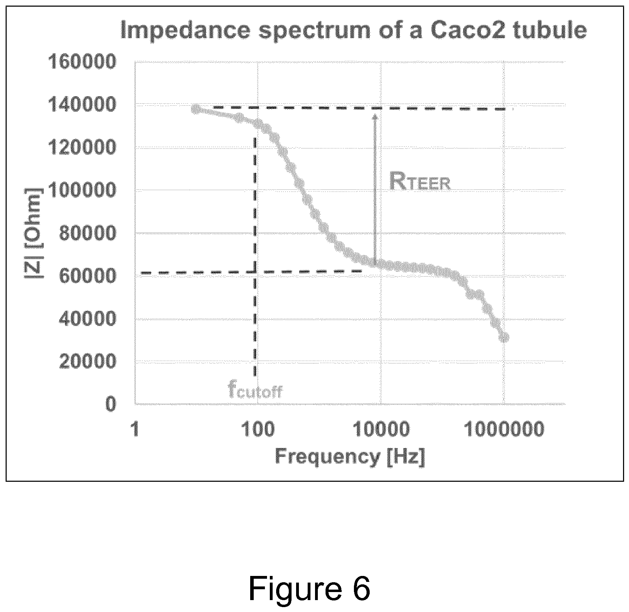

Figures 6 to 8 are discussed in the examples below.

### Examples

Day 0: Collagen-1 gel was injected into one of the channels (gel channel) of a 2-lane Organoplate. After curing of the gel, Caco-2 cells (Sigma, human colon carcinoma cells) in culture medium were seeded in the other channel (perfusion channel) and allowed to proliferate. Culture medium was refreshed daily. Within 4 days a tubule with an apical side (perfusion channel) and a basolateral side (gel channel) had been formed. Following this protocol, 40 chips, comprising a gel channel and a perfusion channel separated by a phaseguide, were seeded with Caco-2 cells, meaning that 40 tubules were grown simultaneously in a single 2-lane Organoplate. The method of culturing cells in this system is similar to the one described by Trietsch et al (Nature Communications, volume 8, Article number: 262(2017), doi:10.1038/s41467-017-00259-3).

A TEER measurement was performed daily at days 1 to 11. To this end, gold plated electrodes were inserted into the access wells of the gel and perfusion channel. For 5 to 10 seconds per chip, impedance spectra were recorded at frequencies ranging logarithmically from 10Hz to 1MHz.

Figure 6 shows a typical impedance spectrum of a Caco-2 tubule. As shown in the Figure, from this spectrum the TEER value can be derived, optionally by fitting the data to a theoretical model.

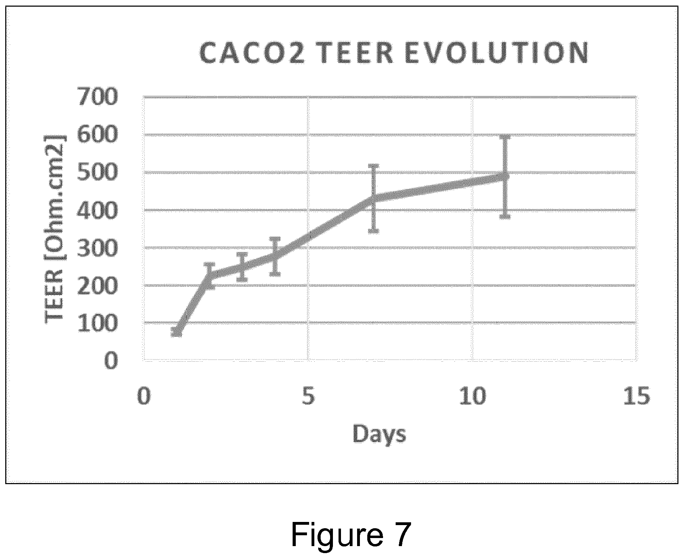

Figure 7 shows the evolution of the barrier resistance of the Caco-2 cells over time. TEER values were extracted from obtained impedance spectra. A higher TEER corresponds to an increased barrier function.

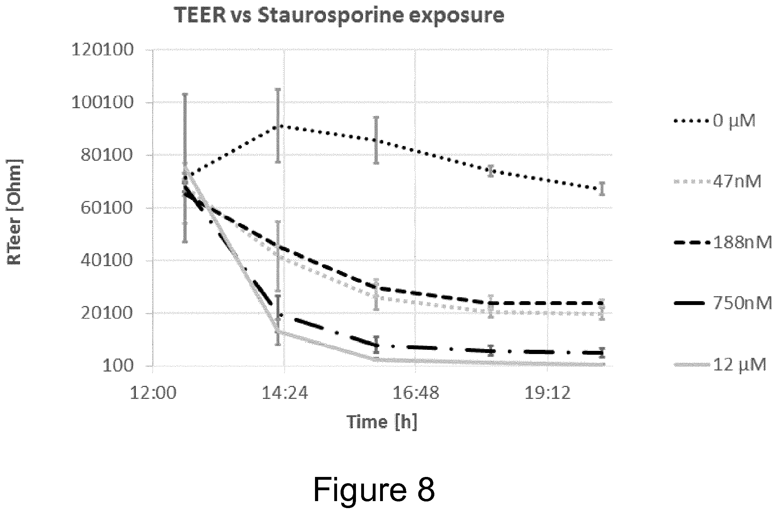

At day 5, the 40 Caco-2 tubules were exposed to different concentrations of staurosporine (Sigma, S4400) for 12 hours. Figure 8 shows the evolution of the TEER over time during exposure to different concentrations of staurosporine. As can be seen, there is a concentration dependent effect on the TEER values. Even at a concentration of 47 nM, an effect on the TEER can be observed, far lower than what is detectable with diffusion-based techniques using fluorescent microscopy.

The above description is for the purpose of teaching the person of ordinary skill in the art how to practice the present invention, and it is not intended to detail all those modifications and variations which will become apparent upon reading the description. It is intended, however, that all such modifications and variations be included within the scope of the present invention, which is defined by the following claims.

## Claims

1. A device for performing electrical measurements, comprising:
a cassette having first and second surfaces, the cassette configured to engage with a microtiter plate and comprising a plurality of electrodes extending from the first surface in the direction of the microtiter plate when the cassette is engaged with the microtiter plate; and
a housing detachably attached to the second surface of the cassette, the housing comprising one or more heat management elements, and a processor comprising a data acquisition module electrically connected to the electrodes and a data processing module.

2. A device according to claim 1, wherein the device is configured for impedance spectroscopy, potentiometry, voltammetry or amperometry and/or wherein the device is configured for measuring transepithelial or transendothelial electrical resistance (TEER).

3. A device according to any one of the preceding claims, wherein the processor is configured to perform AC frequency sweeps, preferably in a range of from 1 Hz to 100 Mhz, more preferably in a range of from 10 Hz to 10 MHz, preferably wherein the range of the frequency sweep and frequency of data acquisition by the data acquisition module is adaptable in a manual, automated or iterative fashion, preferably optimized to the characteristics of the system being measured.

4. A device according to any one of the preceding claims, wherein the plurality of electrodes is disposed in a predetermined configuration corresponding to the configuration of at least two or more wells of a microtiter plate; wherein the microtiter plate preferably comprises 96 microfluidic chips and wherein the microtiter plate preferably is a 384 well plate complying to the ANSI SLAS standards 1 to 4-2004.

5. A device according to claim 4, wherein the configuration of the electrodes is such that at least one subset of the electrodes is configured to correspond to at least one subset of wells that are microfluidically connected in the microtiter plate and wherein the electrodes are configured to be immersed in a fluid inside the wells, thus incorporating the fluid in the electrical circuit.

6. A device according to claim 5, wherein each subset of electrodes contains at least a load, sense and reference electrode.

7. A device according to claim 5 or 6, wherein each subset of electrodes contains two or more electrodes that are directly connected in the electrical circuit, and wherein said two or more electrodes are connected to one or more wells of the same microfluidic channel to reduce the effective electrical resistance of the channel, preferably wherein 2 or more of said subset of electrodes are configured such that the electrical circuit, formed when the cassette is engaged with the microtiter plate, has similar electrical resistance across the directly connected electrodes, preferably minimizing the effect of the position/localization of local differences in electrical characteristics on the apparent electrical characteristics of the electrical circuit.

8. A device according to any one of the preceding claims, wherein two or more electrodes are immersed into a single well, thereby allowing for a 4-point electrical measurement which enables better electrical characterization of the electrical circuit and/or device under test (DUT).

9. A device according to any one of the preceding claims, wherein the electrode material comprises a biocompatible material, wherein the electrode material preferably is platinum, gold plated brass, gold plated stainless steel or stainless steel.

10. A device according to any one of the preceding claims, wherein the electrodes comprise one or more of a silver chloride electrode, an ion selective electrode, or a biofunctionalized electrode.

11. A device according to any one of the preceding claims, wherein the one or more heat management elements comprises elements thermally decoupling the cassette from the housing, such as insulating layers or spacers between the housing and cassette, wherein the one or more heat management elements includes passive or active heat conduits to move heat away from the cassette and wherein the one or more heat management elements comprise one or more of radiating surfaces, cooling fins, liquid cooling, Peltier modules, air ducts, or fans improving airflow through or around the device, or any combination thereof.

12. An *in vitro* method for measuring electrical properties of a layer of cells cultured in a microfluidic device, the method comprising the steps of
a. providing a microfluidic device comprising a plurality of microfluidic channels, wherein at least one of the microfluidic channels is filled at least in part with a gel; and wherein at least one of the microfluidic channels comprises cells as a layer on or against the gel with an apical and a basolateral side, preferably the layer of cells having a tubular structure with an apical and a basolateral side in the microfluidic channel;
b. providing to the microfluidic channels at least one electrode in connection with the fluid in contact with the apical side and at least one electrode in connection with the fluid in contact with the basolateral side; thus incorporating the microfluidic channel in the electrical circuit;
c. measuring the impedance spectrum, voltage or current, wherein the microfluidic device is a microtiter plate, and
wherein the method is performed using the device of any one of claims 1 to 11.

13. The method according to claim 12, wherein the gel is structured in the microfluidic channel by means of capillary pressure techniques, such as pillars, ridges, groves, hydrophobic patches or less hydrophilic patches in a predominantly more hydrophilic channel.

14. The method according to claim 12 or 13, wherein flow is induced through at least a subset of the microfluidic channels during the measurement, wherein said flow is induced by liquid levelling, preferably by reversibly tilting the microfluidic device.

15. The method according to any one of claims 12 to 14, wherein multiple cell layers and/or microfluidic channels are part of the same microfluidic network, and wherein measuring across multiple cell layers occurs in a single, sequential or parallel measurement.

16. The method according to any one of claims 12 to 15, wherein the cultured cells are exposed to one or more compounds or other stimuli before or during the measurement, to observe the effect of said stimuli on the barrier function.

17. The method according to any one of claims 12 to 16, wherein the measurement is performed multiple times to monitor the barrier function over time.

## Patentansprüche

1. Vorrichtung zum Durchführen elektrischer Messungen, umfassend:
eine Kassette mit einer ersten und einer zweiten Oberfläche,
wobei die Kassette zum Eingriff mit einer Mikrotiterplatte konfiguriert ist und eine Vielzahl von Elektroden umfasst, die sich von der ersten Oberfläche in Richtung der Mikrotiterplatte erstreckt, wenn die Kassette mit der Mikrotiterplatte in Eingriff steht; und
ein Gehäuse, das lösbar an der zweiten Oberfläche der Kassette angebracht ist, wobei das Gehäuse ein oder mehrere Wärmemanagementelemente umfasst, und einen Prozessor, der ein Datenerfassungsmodul umfasst, das elektrisch mit den Elektroden verbunden ist, und ein Datenverarbeitungsmodul.

2. Vorrichtung nach Anspruch 1, wobei die Vorrichtung für Impedanzspektroskopie, Potentiometrie, Voltammetrie oder Amperometrie konfiguriert ist und/oder wobei die Vorrichtung zum Messen des transepithelialen oder transendothelialen elektrischen Widerstands (TEER) konfiguriert ist.

3. Vorrichtung nach einem der vorhergehenden Ansprüche, wobei der Prozessor so konfiguriert ist, dass er AC-Frequenz-Sweeps durchführt, vorzugsweise in einem Bereich von 1 Hz bis 100 MHz, mehr bevorzugt in einem Bereich von 10 Hz bis 10 MHz, wobei vorzugsweise der Bereich des Frequenz-Sweeps und die Frequenz der Datenerfassung durch das Datenerfassungsmodul auf manuelle, automatisierte oder iterative Weise anpassbar ist, vorzugsweise optimiert auf die Eigenschaften des zu messenden Systems.

4. Vorrichtung nach einem der vorhergehenden Ansprüche, wobei die Vielzahl von Elektroden in einer vorbestimmten Konfiguration angeordnet ist, die der Konfiguration von mindestens zwei oder mehr Vertiefungen einer Mikrotiterplatte entspricht; wobei die Mikrotiterplatte vorzugsweise 96 mikrofluidische Chips umfasst und wobei die Mikrotiterplatte vorzugsweise eine Platte mit 384 Vertiefungen ist, die den ANSI-SLAS-Standards 1 bis 4-2004 entspricht.

5. Vorrichtung nach Anspruch 4, wobei die Konfiguration der Elektroden derart ist, dass mindestens eine Untergruppe der Elektroden so konfiguriert ist, dass sie mindestens einer Untergruppe von Vertiefungen entspricht, die mikrofluidisch in der Mikrotiterplatte verbunden sind, und wobei die Elektroden so konfiguriert sind, dass sie in eine Flüssigkeit in den Vertiefungen eingetaucht sind, wodurch die Flüssigkeit in den elektrischen Kreislauf eingebunden wird.

6. Vorrichtung nach Anspruch 5, wobei jede Untergruppe von Elektroden mindestens eine Last-, Erfassungs- und Bezugselektrode enthält.

7. Vorrichtung nach Anspruch 5 oder 6, wobei jede Untergruppe von Elektroden zwei oder mehr Elektroden enthält, die direkt mit dem elektrischen Stromkreis verbunden sind, und wobei die zwei oder mehr Elektroden mit einer oder mehreren Vertiefungen desselben mikrofluidischen Kanals verbunden sind, um den effektiven elektrischen Widerstand des Kanals zu reduzieren, wobei vorzugsweise 2 oder mehr der Untergruppe von Elektroden so konfiguriert sind, dass der elektrische Schaltkreis, der gebildet wird, wenn die Kassette mit der Mikrotiterplatte in Eingriff steht, einen ähnlichen elektrischen Widerstand über die direkt verbundenen Elektroden aufweist, wodurch vorzugsweise der Effekt der Position/Lokalisierung lokaler Unterschiede in den elektrischen Eigenschaften auf die scheinbaren elektrischen Eigenschaften des elektrischen Schaltkreises minimiert wird.

8. Vorrichtung nach einem der vorhergehenden Ansprüche, wobei zwei oder mehr Elektroden in eine einzige Vertiefung eingetaucht sind, wodurch eine elektrische 4-Punkt-Messung ermöglicht wird, die eine bessere elektrische Charakterisierung des elektrischen Schaltkreises und/oder der zu testenden Vorrichtung (DUT) ermöglicht.

9. Vorrichtung nach einem der vorhergehenden Ansprüche, wobei das Elektrodenmaterial ein biokompatibles Material umfasst, wobei das Elektrodenmaterial vorzugsweise Platin, vergoldetes Messing, vergoldeter rostfreier Stahl oder rostfreier Stahl ist.

10. Vorrichtung nach einem der vorhergehenden Ansprüche, wobei die Elektroden eine oder mehrere einer Silberchloridelektrode, einer ionenselektiven Elektrode oder einer biofunktionalisierten Elektrode umfassen.

11. Vorrichtung nach einem der vorhergehenden Ansprüche, wobei das eine oder die mehreren Wärmemanagementelemente Elemente umfassen, die die Kassette thermisch von dem Gehäuse entkoppeln, wie etwa Isolierschichten oder Abstandshalter zwischen dem Gehäuse und der Kassette, wobei das eine oder die mehreren Wärmemanagementelemente passive oder aktive Wärmeleitungen enthalten, um Wärme von der Kassette wegzuleiten, und wobei das eine oder die mehreren Wärmemanagementelemente eines oder mehrere von abstrahlenden Oberflächen, Kühlrippen, Flüssigkeitskühlung, Peltier-Modulen, Luftkanälen oder Ventilatoren umfassen, die den Luftstrom durch oder um die Vorrichtung verbessern, oder eine beliebige Kombination davon.

12. In-vitro-Verfahren zum Messen elektrischer Eigenschaften einer Zellschicht, die in einer mikrofluidischen Vorrichtung kultiviert wird, wobei das Verfahren die folgenden Schritte umfasst:
a. Bereitstellen einer mikrofluidischen Vorrichtung, die eine Vielzahl von mikrofluidischen Kanälen umfasst, wobei mindestens einer der mikrofluidischen Kanäle mindestens teilweise mit einem Gel gefüllt ist; und wobei mindestens einer der mikrofluidischen Kanäle Zellen als eine Schicht auf oder gegen das Gel mit einer apikalen und einer basolateralen Seite umfasst, wobei vorzugsweise die Zellschicht eine röhrenförmige Struktur mit einer apikalen und einer basolateralen Seite in dem mikrofluidischen Kanal aufweist;
b. Bereitstellen mindestens einer Elektrode in Verbindung mit dem Fluid in Kontakt mit der apikalen Seite und mindestens einer Elektrode in Verbindung mit dem Fluid in Kontakt mit der basolateralen Seite an den mikrofluidischen Kanälen; wodurch der mikrofluidische Kanal in den elektrischen Schaltkreis eingebaut wird;
c. Messen des Impedanzspektrums, der Spannung oder des Stroms, wobei die mikrofluidische Vorrichtung eine Mikrotiterplatte ist, und
wobei das Verfahren unter Verwendung der Vorrichtung nach einem der Ansprüche 1 bis 11 durchgeführt wird.

13. Verfahren nach Anspruch 12, wobei das Gel in dem mikrofluidischen Kanal mittels Kapillardrucktechniken strukturiert wird, wie Säulen, Grate, Rillen, hydrophoben Patches oder weniger hydrophilen Patches in einem überwiegend hydrophileren Kanal.

14. Verfahren nach Anspruch 12 oder 13, wobei während der Messung eine Strömung durch mindestens eine Untergruppe der mikrofluidischen Kanäle induziert wird, wobei die Strömung durch Flüssigkeitsnivellierung, vorzugsweise durch reversibles Neigen der mikrofluidischen Vorrichtung, induziert wird.

15. Verfahren nach einem der Ansprüche 12 bis 14, wobei mehrere Zellschichten und/oder mikrofluidische Kanäle Teil desselben mikrofluidischen Netzwerks sind, und wobei das Messen über mehrere Zellschichten hinweg in einer einzigen, sequentiellen oder parallelen Messung erfolgt.

16. Verfahren nach einem der Ansprüche 12 bis 15, wobei die kultivierten Zellen vor oder während der Messung einer oder mehreren Verbindungen oder anderen Stimuli ausgesetzt werden, um die Wirkung der Stimuli auf die Barrierefunktion zu beobachten.

17. Verfahren nach einem der Ansprüche 12 bis 16, wobei die Messung mehrere Male durchgeführt wird, um die Barrierefunktion über die Zeit zu überwachen.

## Revendications

1. Dispositif pour effectuer des mesures électriques, comprenant :
une cassette ayant des première et seconde surfaces, la cassette étant configurée pour venir en prise avec une plaque de microtitration et comprenant une pluralité d'électrodes s'étendant depuis la première surface dans la direction de la plaque de microtitration lorsque la cassette est en prise avec la plaque de microtitration ; et
un boîtier fixé de manière amovible à la seconde surface de la cassette, le boîtier comprenant un ou plusieurs éléments de gestion thermique, et un processeur comprenant un module d'acquisition de données connecté électriquement aux électrodes et un module de traitement de données.

2. Dispositif selon la revendication 1, dans lequel le dispositif est configuré pour la spectroscopie d'impédance, la potentiométrie, la voltammétrie ou l'ampérométrie et/ou dans lequel le dispositif est configuré pour mesurer la résistance électrique transépithéliale ou transendothéliale (TEER).

3. Dispositif selon l'une quelconque des revendications précédentes, dans lequel le processeur est configuré pour effectuer des balayages de fréquence CA, de préférence dans une plage de 1 Hz à 100 MHz, plus préférablement dans une plage de 10 Hz à 10 MHz, de préférence dans lequel la plage du balayage de fréquence et la fréquence d'acquisition de données par le module d'acquisition de données est adaptable de manière manuelle, automatisée ou itérative, de préférence optimisée aux caractéristiques du système mesuré.

4. Dispositif selon l'une quelconque des revendications précédentes, dans lequel la pluralité d'électrodes est disposée dans une configuration prédéterminée correspondant à la configuration d'au moins deux puits ou plus d'une plaque de microtitration ; dans lequel la plaque de microtitration comprend de préférence 96 puces microfluidiques et dans lequel la plaque de microtitration est de préférence une plaque à 384 puits conforme aux normes ANSI SLAS 1 à 4-2004.

5. Dispositif selon la revendication 4, dans lequel la configuration des électrodes est telle qu'au moins un sous-ensemble des électrodes est configuré pour correspondre à au moins un sous-ensemble de puits qui sont connectés de manière microfluidique dans la plaque de microtitration et dans lequel les électrodes sont configurées pour être immergées dans un fluide à l'intérieur des puits, incorporant ainsi le fluide dans le circuit électrique.

6. Dispositif selon la revendication 5, dans lequel chaque sous-ensemble d'électrodes contient au moins une électrode de charge, de détection et de référence.

7. Dispositif selon la revendication 5 ou 6, dans lequel chaque sous-ensemble d'électrodes contient deux électrodes ou plus qui sont directement connectées dans le circuit électrique, et dans lequel lesdites deux électrodes ou plus sont connectées à un ou plusieurs puits du même canal microfluidique pour réduire la résistance électrique effective du canal, de préférence dans lequel 2 ou plus dudit sous-ensemble d'électrodes sont configurées de telle sorte que le circuit électrique, formé lorsque la cassette est en prise avec la plaque de microtitration, a une résistance électrique similaire à travers les électrodes directement connectées, minimisant de préférence l'effet de la position/l'emplacement des différences locales de caractéristiques électriques sur les caractéristiques électriques apparentes du circuit électrique.

8. Dispositif selon l'une quelconque des revendications précédentes, dans lequel deux électrodes ou plus sont immergées dans un seul puits, permettant ainsi une mesure électrique en 4 points qui permet une meilleure caractérisation électrique du circuit électrique et/ou dispositif sous test (DUT).

9. Dispositif selon l'une quelconque des revendications précédentes, dans lequel le matériau d'électrode comprend un matériau biocompatible, dans lequel le matériau d'électrode est de préférence du platine, du laiton plaqué or, de l'acier inoxydable plaqué or ou de l'acier inoxydable.

10. Dispositif selon l'une quelconque des revendications précédentes, dans lequel les électrodes comprennent une ou plusieurs parmi une électrode de chlorure d'argent, une électrode sélective d'ions ou une électrode biofonctionnalisée.

11. Dispositif selon l'une quelconque des revendications précédentes, dans lequel les un ou plusieurs éléments de gestion thermique comprennent des éléments découplant thermiquement la cassette du boîtier, tels que des couches isolantes ou des entretoises entre le boîtier et la cassette, dans lequel les un ou plusieurs éléments de gestion thermique comportent des conduits de chaleur passifs ou actifs pour éloigner la chaleur de la cassette et dans lequel les un ou plusieurs éléments de gestion thermique comprennent un ou plusieurs parmi des surfaces rayonnantes, des ailettes de refroidissement, un refroidissement par liquide, des modules Peltier, des conduits d'air ou des ventilateurs améliorant le flux d'air à travers ou autour du dispositif, ou toute combinaison de ceux-ci.

12. Procédé *in vitro* pour mesurer des propriétés électriques d'une couche de cellules cultivées dans un dispositif microfluidique, le procédé comprenant les étapes de
a. fourniture d'un dispositif microfluidique comprenant une pluralité de canaux microfluidiques, dans lequel au moins l'un des canaux microfluidiques est rempli au moins en partie d'un gel ; et dans lequel au moins l'un des canaux microfluidiques comprend des cellules en tant que couche sur ou contre le gel avec un côté apical et un côté basolatéral, de préférence la couche de cellules ayant une structure tubulaire avec un côté apical et un côté basolatéral dans le canal microfluidique ;
b. fourniture aux canaux microfluidiques d'au moins une électrode en connexion avec le fluide en contact avec le côté apical et au moins une électrode en connexion avec le fluide en contact avec le côté basolatéral ; incorporant ainsi le canal microfluidique dans le circuit électrique ;
c. mesure du spectre d'impédance, de la tension ou du courant, dans lequel le dispositif microfluidique est une plaque de microtitration, et
dans lequel le procédé est exécuté en utilisant le dispositif selon l'une quelconque des revendications 1 à 11.

13. Procédé selon la revendication 12, dans lequel le gel est structuré dans le canal microfluidique au moyen de techniques de pression capillaire, telles que des piliers, des crêtes, des rainures, des patchs hydrophobes ou des patchs moins hydrophiles dans un canal majoritairement plus hydrophile.

14. Procédé selon la revendication 12 ou 13, dans lequel l'écoulement est induit à travers au moins un sous-ensemble des canaux microfluidiques pendant la mesure, dans lequel ledit écoulement est induit par un nivellement de liquide, de préférence par une inclinaison réversible du dispositif microfluidique.

15. Procédé selon l'une quelconque des revendications 12 à 14, dans lequel plusieurs couches de cellules et/ou canaux microfluidiques font partie du même réseau microfluidique, et dans lequel la mesure à travers de multiples couches de cellules se produit dans une mesure unique, séquentielle ou parallèle.

16. Procédé selon l'une quelconque des revendications 12 à 15, dans lequel les cellules cultivées sont exposées à un ou plusieurs composés ou autres stimuli avant ou pendant la mesure, pour observer l'effet desdits stimuli sur la fonction de barrière.

17. Procédé selon l'une quelconque des revendications 12 à 16, dans lequel la mesure est effectuée plusieurs fois pour surveiller la fonction de barrière dans le temps.
